(19) Europäisches Patentamt
European Patent Office
Office européen des brevets

(11) Publication number: **0 065 790**
**B1**

(12) **EUROPEAN PATENT SPECIFICATION**

(45) Date of publication of patent specification: **03.07.85**

(21) Application number: **82200466.9**

(22) Date of filing: **16.04.82**

(51) Int. Cl.⁴: **C 07 C 101/02,**
A 61 K 31/195, A 61 K 31/22

(54) **Compounds with GABA-like activity, process for their preparation and pharmaceutical compositions containing them.**

(30) Priority: **26.05.81 IT 2196081**

(43) Date of publication of application:
**01.12.82 Bulletin 82/48**

(45) Publication of the grant of the patent:
**03.07.85 Bulletin 85/27**

(84) Designated Contracting States:
**AT BE CH DE FR GB LI LU NL SE**

(56) References cited:
**BULLETIN OF THE CHEMICAL SOCIETY OF JAPAN, vol. 66, no. 2, February 1963, pages 179-183; S. MOROSAWA et al.: "Synthesis and pyrolysis of N-Substituted gamma-Aminobutyrates and N-Substituted Bis(beta-ethoxycarbonylethyl)amines"**

**CHEMICAL ABSTRACTS, vol. 90, no. 5, 29th January 1979, page 521, no. 39227n, Columbus Ohio (USA); C. ANSELMI et al.: "New synthesis of a natural amino acid (N-(4-aminobutyl)-3-aminopropioni c acid) - its possible cosmetic and dermopharmaceutical applications"**

(73) Proprietor: **HEPAR CHIMIE S.A.
Grand Places, 14
Fribourg (CH)**

(72) Inventor: **Curti, Maria
Via Riviera, 52
I-27020 Torre d'Isola Pavia (IT)**
Inventor: **Fussi, Fernando
Via Foscolo, 31
I-20050 Lesm, Milano (IT)**

(74) Representative: **Appoloni, Romano et al
Ing. Barzanò & Zanardo S.p.A. Via Borgonuovo 10
I-20121 Milano (IT)**

(56) References cited:
**CHEMICAL ABSTRACTS, vol. 90, no. 17, 23rd April 1979, page 453, no. 137153e, Columbus Ohio (USA); B. HINTZE et al.: "Stereoselectivity of the carbon-nitrogen bond formation. III. Addition of racemic and chiral amines to crotononitrile and methacrylonitrile"**

**EP 0 065 790 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The physiological and metabolic part played by gamma-amino butyric acid (GABA) in inhibitory synaptic transmission in the CNS is known.

Any decrease in the cerebral levels of GABA caused by blockade of or congenital defects affecting the cerebral enzyme glutamic dehydrogenase (GLDH), which regulates the biosynthesis of GABA starting from glutamic acid, causes increased excitability of the nerve cells, with onset of a convulsive state. Conditions of cerebral GABA-depletion are obtained experimentally by administering semicarbizide or thiosemicarbizide (GLDH inhibitors) or other inhibitors, by induced Vitamin $B_6$ deficiencies, insulin shock, alkalosis.

In pathological conditions, decrease in cerebral levels of GABA causes convulsions and atonic or hypotonic loss of consciousness typical of epileptic seizures ("grand mal" and "petit mal").

Other neuropathological conditions are also, at least in part, ascribable to metabolic derangements of cerebral GABA. One problem involved in the use of GABA is connected with the fact that the substance does not cross the blood-brain barrier and consequently, when administered either orally or parenterally, does not reach the cerebral districts, or does so only to a minimal extent. A first object of the invention is to solve this problem. In this regard, according to the present invention, it has now been surprisingly found that the compounds of the formula given below possess a GABA-like anti-convulsant activity:

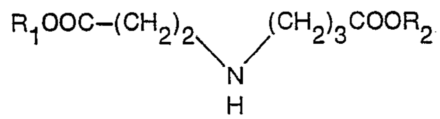

(I)

wherein:

$R_1 = R_2 =$ H in the free form, corresponding to N-β-carboxyethyl-γ-amino butyric acid,

$R_1 =$ H; $R_2 =$ alkyl radical; or

$R_1 =$ alkyl radical; $R_2 =$ H (monoesters)

$R_1$ and $R_2 =$ alkyl radical (diesters)

and that said compounds, when administered parenterally, cross the blood-brain barrier and have said GABA-like effect in the CNS. According to the invention, this effect has for example been demonstrated by the protracting of the latency period of experimental convulsions induced by insoniazid and semicarbizide. The biochemical mechanism by which these substances act on reaching the brain is probably an enzymatic dissociation of the molecule, with liberation of active GABA.

Such mechanism clearly makes it possible to by-pass the metabolic blockade — occurring in given pathological conditions — of the biosynthesis of GABA starting from the glutamic acid from the physiological amino-acid pool. N-β-carboxyethyl-γ-amino butyric acid (hereinafter referred to as CEGABA) is known in the literature of organic synthesis, and in Nippon Kagak Zasshi *77,* 599—602 (1956) Akira Yokoo and Shiro Morosawa have described its synthesis. The corresponding ethyl diester is also known from Bulletin of the Medical Society of Japan, Volume 66, No. 2, February 1963, pages 179—183; S. Morosana et al; "Synthesis and Pyrolysis of N-substituted alpha-aminobutyrates and N-substituted bis (betaethoxycarbonylethyl) amines".

There are, however, certain problems connected with this known synthesis; in particular it would be most desirable to obtain with it final products of greater purity, with higher processing yields and using a smaller number of reaction steps. A further object of the present invention is therefore to provide a process for the synthesis of compounds having GABA-like activity of said general formula (I), such as will obtain the aforesaid desirable advantages as compared to the known art.

To attain this object the present invention proposes a process for the preparation of said compounds of general formula:

$$R_1OOC{-}(CH_2)_2{-}NH{-}(CH_2)_3{-}COOR_2 \qquad (I)$$

wherein $R_1$ and $R_2 =$ H or a lower alkyl, characterized in that it comprises a condensation of a substrate selected from: gamma-amino butyric acid (II) (or GABA), its lactam pyrrolidine-2-one (II)', or its esters, with a reagent selected from: acrylonitrile, acrylic acid, or its esters.

Schematic reaction diagrams are given below relating to possible preferred forms of execution of the process of the invention as substantially defined above.

Schematic diagram A —

Condensation of gamma-amino butyric acid (II) with acrylonitrile (III), followed by hydrolysis to give CEGABA (V)

$$H_2N-(CH_2)_3-COOH \quad + \quad CH_2=CHCN \longrightarrow$$

(II) (III)

$$NC(CH_2)_2\,NH-(CH_2)_3-COOH \xrightarrow{H_2O}$$

(IV)

$$COOH-(CH_2)_3-NH-(CH_2)_2-COOH$$

(V)

Schematic diagram A' —

Condensation according to diagram A, starting from the lactam pyrrolidine-2-one (II)' with opening of the ring, to give (II)

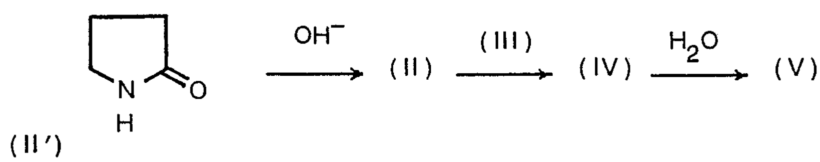


Schematic diagram B —

Condensation of (II) with acrylic acid (VI) or with one of its esters (VII), to give respectively CEGABA (V) or one of its monoesters (VIII).

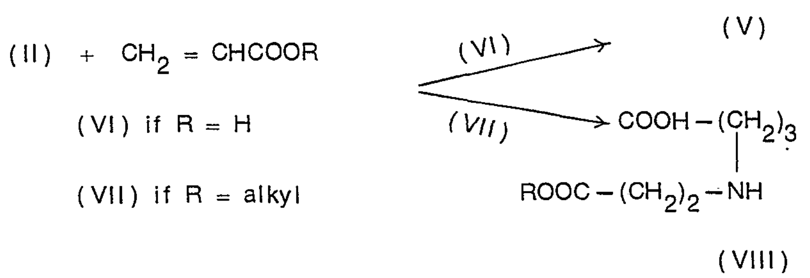


Schematic diagram C —

Esterification of (II) to give the ester (XI), and subsequent condensation with (VI) or with (VII), to give respectively the monoester (IX) or the diester (X) of CEGABA

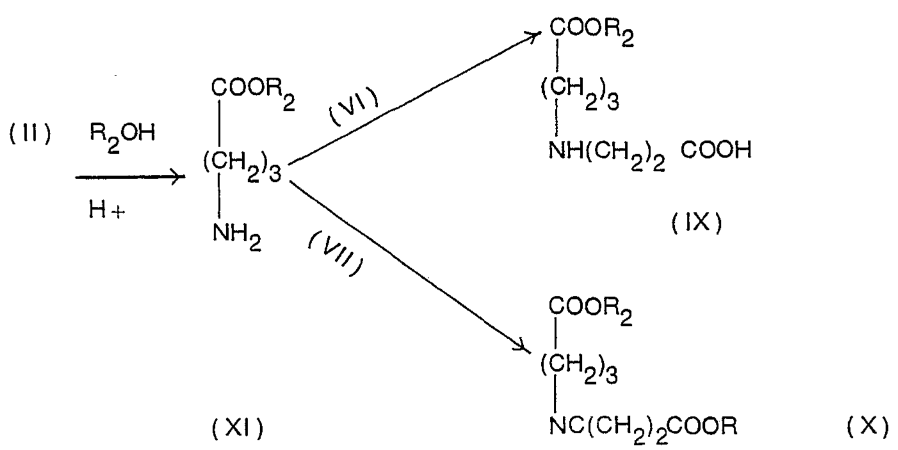

Schematic diagram D —

Esterification of CEGABA (V) to give the corresponding diester

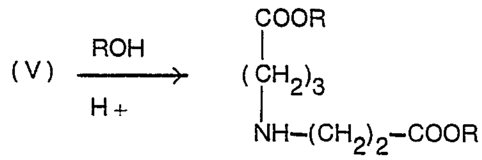


R = alkyl (I) wherein R = alkyl

Some non-restrictive examples of synthesis as provided for by the process according to the invention are given below.

Example 1

passage from the compound (II)′ to (II).

A quantity of 15.5 cc of pyrrolidine-2-one (II)′ was refluxed for one hour with 20 cc of a 40% solution of NaOH.

The whole was cooled rapidly and the product (II) was used directly for the subsequent reaction.

Storage: at 0°C, for 2 or 3 days.

Example 2

from II to XI

A quantity of 10.3 g of GABA (II) in 100 cc ethanol saturated with gaseous HCl was refluxed for 6 hours.

The whole was evaporated to dryness; the ester (XI) was used as such for the subsequent reactions.

Example 3

from II to V via IV

A quantity of 51.5 g of GABA (II) was suspended in 50 cc of water, IRA 400 was added to adjust to a strongly basic pH. Under agitation, and gently in order not to exceed 10°C, addition was made of 30 g of acrylonitrile (III). The whole was allowed to react at room temperature for 3 hours, after which it was filtered and precipitated from acetone.

The crude product was introduced into a silica gel column and eluted with increasingly polar mixtures of $CH_2Cl_2$: MeOH.

The pure product isolated in this way was crystallized from methanol, to obtain 43 g of N-β-cyanoethyl-γ-amino butyric acid (IV), melting range 122—124°C.

A quantity of 10 g of (IV) was dissolved in 50 cc of 37% HCl and heated at reflux for 1 hour. The whole was then treated with anionic resin until there was no longer any presence of chlorides, and then with IRC 50 to pH 3.5. Freeze-drying was then performed.

There were obtained 9.5 g of N-β-carboxyethyl-γ-amino butyric acid (V); melting range 149—151°C.

Example 4

from II to V

A quantity of 51.5 g of GABA (II) was dissolved in 150 cc of 20% NaOH. At a temperature not exceeding 10°C, slow addition was made of 45 g of acrylic acid. The whole was left to react for 3 hours at room temperature and then brought to 70°C, this temperature being held for 12 hours. The solution was treated with IRC 50 to pH 3.5. The crude product was obtained by freeze-drying from a silica gel column, operating as described in Example 3, to obtain the pure product which crystallized from methanol. There were obtained 20 g of N-β-carboxyethyl-γ-amino butyric acid (V), melting range: 148—151°C.

The non-reacted gamma-amino butyric acid (II) was eluted from the column with pure methanol.

Example 5

from II to VIII

A quantity of 51.5 g of GABA (II) was dissolved in 500 cc of MeOH containing 13 g of Na. At a temperature not in excess of 10°C, slow addition was made of 65 g of ethyl acrylate (VII) in 300 cc of MeOH. The whole was then heated at reflux for 5 hours.

After evaporation of most of the solvent, the residue was taken up with $CH_2Cl_2$ and introduced into a silica gel column. The fraction containing the product of interest was concentrated and the product crystallized from MeOH by addition of HCl gas. There were obtained 18 g of (VIII)·HCl.

Example 6

from XI to IX

The product (XI) obtained in Example 2 was added to 100 cc of MeOH containing 2 g of Na.

Slow addition was made of 9 g of acrylic acid (VI) in 50 cc of MeOH, at a temperature of not more than 10°C. the whole was allowed to stand at room temperature for 12 hours. After evaporation of most of the solvent the product (IX) was taken up with $CH_2Cl_2$ and introduced into a silica gel column.

The fraction containing the product of interest was concentrated and the product crystallized from MeOH both as such and as hydrochloride.

Example 7

from XI to X

The product (XI) obtained in Example 2 was treated in the same conditions as in Example 6 with 13 g of ethyl acrylate (VII). The product (X) crystallized from MeOH both as such and as hydrochloride.

Example 8

from V to X

A quantity of 10 g of CEGABA (V) in 100 cc of EtOH saturated with HCl gas was refluxed for 6 hours. On concentration in *vacuo,* the product (X) ($R_1 = R_2 =$ EtOH) crystallized as hydrochloride. The cyanoethylation of amines with acrylonitrile and the condensation of acrylic acid, or its esters, on amines are known basic processes in organic synthesis (see for instance Kirk-Othmer, Encyclopedia of Chemical Technology, 3rd Ed. Volume 7, page 376, Table 3 "Cyanoethylation on Nitrogen", Chemical Abstracts, Volume 90, No 5, 29 Jan 1979, page 521 No. 39227n. C. Anselm et al: "New synthesis of a natural amino acid (N-(4-aminobutyl)-3-aminopropionic acid)- its possible cosmetic and dermopharmaceutical applications. Riv. Ital. Essenze, Profumi, Piante off., Aromet, Syndets, Saponi, Cosmet, Aerosols *1978,* 60 (4), 245—6. "Abstract", Bulletin of the Medical Society of Japan, Volume 66, No. 2, February 1963, pages 179—183; S. Morosana et al; "Synthesis and Pyrolysis of N-substituted alpha-aminobutyrates and N-substituted bis (betaethoxycarbonylethyl) amines").

The hydrolysis of a monocyanoethylated derivative to give the corresponding carboxylic acid is also well known (e.g. see the reference to Anselm et al mentioned above).

As stated, the compounds of formula (I) of the invention can be used advantageously as pharmaceuticals, in suitable compositions in which said compounds are contained as active principles.

Preferred dosages are the following:

| Administration | dosage/day |
|---|---|
| oral | 4—20 mg/kg |
| parenteral | 1—10 mg/kg |
| rectal | 2—20 mg/kg |

**Claims for the Contracting States: BE CH DE FR GB LI LU NL SE**

1. Compounds possessing GABA-like anti-convulsant activity in the brain, characterized in that they are of general formula:

$$R_1OOC—(CH_2)_2—NH—(CH_2)_3—COOR_2 \qquad (I)$$

wherein $R_1$ and $R_2$ = H or lower alkyl, with the proviso that when $R_1$ (or $R_2$) is hydrogen, $R_2$ (or $R_1$) is different from hydrogen, and the proviso that when $R_1$ (or $R_2$) is ethyl, $R_2$ (or $R_1$) is different from ethyl.

2. A process for preparing compounds according to claim 1, characterized in that it comprises a condensation of a substrate selected from: gamma-amino butyric acid (II) (or GABA), or its esters, with a reagent selected from: acrylonitrile, acrylic acid, or its esters.

3. A process according to claim 2, characterized in that, when said reagent is acrylonitrile, said condensation is followed by hydrolysis to obtain said compounds of formula (I).

4. A process according to claim 2, characterized in that the substrate is formed by employing the lactam pyrrolidine-2-one (II)' and opening the lactam ring.

5. A process according to claim 2, characterized in that it comprises a condensation of GABA (II) with acrylic acid, or one of its esters, to give respectively CEGABA (V) or one of its monoesters.

6. A process according to claim 2, characterized in that it comprises the esterification of GABA (II) to give one of its esters (XI), and subsequent condensation with acrylic acid, or one of its esters, to give respectively the monoester (IX) or the diester (X) of CEGABA.

7. A process according to claim 3, characterized in that it comprises the condensation of GABA (II) with acrylonitrile, followed by hydrolysis to give CEGABA (V).

8. A process according to claim 1, characterized in that it comprises an esterification of CEGABA (V) to give the corresponding diester of formula (I) wherein $R_1$ and $R_2$ are alkyls.

9. Compounds of formula (I) where $R_1$ and $R_2$ = H or lower alkyl, to be used to provide a GABA-like activity in the brain.

10. A pharmaceutical composition containing a compound according to claim 9, adapted to provide a GABA-like activity in the brain.

**Claims for the Contracting State: AT**

1. A process for preparing compounds possessing GABA-like anti convulsant activity in the brain of general formula

$$R_1OOC—(CH_2)_2—NH—(CH_2)_3—COOR_2 \quad (I)$$

wherein $R_1$ and $R_2$ = H or lower alkyl, characterized in that it comprises a condensation of a substrate selected from: gamma-amino butyric acid (II) (or GABA), or its esters, with a reagent selected from: acrylonitrile, acrylic acid, or its esters.

2. A process according to claim 1, characterized in that, when said reagent is acrylonitrile, said condensation is followed by hydrolysis to obtain said compounds of formula (I).

3. A process according to claim 1, characterized in that the substrate is formed by employing the lactam pyrrolidine-2-one (II)' and opening the lactam ring.

4. A process according to claim 1, characterized in that it comprises a condensation of GABA (II) with acrylic acid, or one of its esters, to give respectively CEGABA (V) or one of its monoesters.

5. A process according to claim 1, characterized in that it comprises the exterification of GABA (II) to give one of its esters (XI), and subsequent condensation with acrylic acid, or one of its esters, to give respectively the monoester (IX) or the diester (X) of CEGABA.

6. A process according to claim 2, characterized in that it comprises the condensation of GABA (II) with acrylonitrile, followed by hydrolysis to give CEGABA (V).

7. A process according to claim 1, characterized in that it comprises an esterification of CEGABA (V) to give the corresponding diester of formula (I) wherein $R_1$ and $R_2$ are alkyls.

**Patentansprüche: für die Vertragsstaaten: BE CH DE FR GB LI LU NL SE**

1. Verbindungen mit GABA-ähnlicher krampflösender Wirkung im Gehirn, dadurch gekennzeichnet, daß sie die allgemeine Formel

$$R_1OOC—(CH_2)_2—NH—(CH_2)_3—COOR_2 \quad (I)$$

aufweisen, worin $R_1$ und $R_2$ = H oder eine niedere Alkylgruppe sind, mit der Bedingung, daß wenn $R_1$ (oder $R_2$) Wasserstoff ist, $R_2$ (oder $R_1$) verschieden von Wasserstoff ist, und mit der Bedingung, daß wenn $R_1$ (oder $R_2$) Ethyl ist, $R_2$ (oder $R_1$) verschieden von Ethyl ist.

2. Verfahren zur Herstellung von Verbindungen nach Anspruch 1, dadurch gekennzeichnet, daß es die Kondensation eines aus γ-Amino-buttersäure (II) (oder GABA) oder ihrer Ester ausgewählten Substrates mit einem aus Acrylnitril, Acrylsäure oder ihrer Ester ausgewählten Reagens umfaßt.

3. Verfahren nach Anspruch 2, dadurch gekennzeichnet, daß wenn das Reagens Acrylnitril ist, die Kondensation von Hydrolyse zu Verbindungen der Formel (I) gefolgt wird.

4. Verfahren nach Anspruch 2, dadurch gekennzeichnet, daß das Substrat durch Verwendung des Lactams Pyrrolidin-2-on (II)' und Öffnen des Lactamringes gebildet wird.

5. Verfahren nach Anspruch 2, dadurch gekennzeichnet, daß es die Kondensation von GABA (II) mit Acrylsäure oder einem ihrer Ester zu CEGABA (V) bzw. einem seiner Monoester umfaßt.

6. Verfahren nach Anspruch 2, dadurch gekennzeichnet, daß es die Veresterung von GABA (II) zu einem seiner Ester (XI) und anschließende Kondensation mit Acrylsäure oder einem ihrer Ester zum Monoester (IX) bzw. Diester (X) von CEGABA umfaßt.

7. Verfahren nach Anspruch 3, dadurch gekennzeichnet, daß es die Kondensation von GABA (II) mit Acrylnitril und anschließende Hydrolyse zu CEGABA (V) umfaßt.

8. Verfahren nach Anspruch 1, dadurch gekennzeichnet, daß es die Veresterung von CEGABA (V) zum entsprechenden Diester der Formel (I) umfaßt, worin $R_1$ und $R_2$ Alkyl sind.

9. Verbindungen der Formel (I), worin $R_1$ und $R_2$ = H oder niederes Alkyl sind, zur Anwendung zur Entfaltung einer GABA-ähnlichen Wirkung im Gehirn.

10. Eine, eine Verbindung nach Anspruch 1 enthaltende pharmazeutische Zusammensetzung zur Entfaltung einer GABA-aehnlichen Wirkung im Gehirn.

**Patentansprüche: für den Vertragsstaat: AT**

1. Verfahren zur Herstellung von Verbindungen mit GABA-ähnlicher krampflösender Wirkung im Gehirn der allgemeinen Formel

$$R_1OOC—(CH_2)_2—NH—(CH_2)_3—COOR_2 \quad (I)$$

worin $R_1$ und $R_2$ = H oder eine niedere Alkylgruppe sind, dadurch gekennzeichnet, daß es die Kondensation eines aus γ-Amino-buttersäure (II) (oder GABA) oder ihrer Ester ausgewählten Substrates mit einem aus Acrylnitril, Acrylsäure oder ihrer Ester ausgewählten Reagens umfaßt.

2. Verfahren nach Anspruch 1, dadurch gekennzeichnet, daß wenn das Reagens Acrylnitril ist, die Kondensation von Hydrolyse zu Verbindungen der Formel (I) gefolgt wird.

3. Verfahren nach Anspruch 1, dadurch gekennzeichnet, daß das Substrat durch Verwendung des Lactams Pyrrolidin-2-on (II)' und Öffnen des Lactamringes gebildet wird.

4. Verfahren nach Anspruch 1, dadurch gekennzeichnet, daß es die Kondensation von GABA (II) mit Acrylsäure oder einem ihrer Ester zu CEGABA (V) bzw. einem seiner Monoester umfaßt.

5. Verfahren nach Anspruch 1, dadurch gekennzeichnet, daß es die Veresterung von GABA (II) zu einem seiner Ester (XI) und anschließende Kondensation mit Acrylsäure oder einem ihrer Ester zum Monoester (IX) bzw. Diester (X) von CEGABA umfaßt.

6. Verfahren nach Anspruch 2, dadurch gekennzeichnet, daß es die Kondensation von GABA (II) mit Acrylnitril und anschließende Hydrolyse zu CEGABA (V) umfaßt.

7. Verfahren nach Anspruch 1, dadurch gekennzeichnet, daß es die Veresterung von CEGABA (V) zum entsprechenden Diester der Formel (I) umfaßt, worin $R_1$ und $R_2$ Alkyl sind.

**Revendications pour les Etats contractants: BE CH DE FR GB LI LU NL SE**

1. Composés possédant une activité anti-convulsivante, analogue à du GABA, dans le cerveau, caractérisé par le fait qu'ils sont de formule générale:

$$R_1OOC—(CH_2)_2—NH—(CH_2)_3—COOR_2 \quad (I)$$

dans laquelle $R_1$ et $R_2$ = H ou alkyle inférieur, sous réserve que, lorsque $R_1$ (ou $R_2$) est hydrogène, $R_2$ (ou $R_1$) est différent d'hydrogène, et que, lorsque $R_1$ (ou $R_2$) est éthyle, $R_2$ (ou $R_1$) est différent d'éthyle.

2. Procédé de préparation de composés selon la revendication 1, caractérisé par le fait qu'il comprend une condensation d'un substrat choisi parmi: l'acide gamma-aminobutirique (II) (ou GABA) ou ses esters, avec un réactif choisi parmi: acrylonitrile, acide acrylique ou ses esters.

3. Procédé selon la revendication 2, caractérisé par le fait que, lorsque ledit réactif est acrylonitrile, ladite condensation est suivie par une hydrolyse pour obtenir lesdits composés de formule (I).

4. Procédé selon la revendication 2, caractérisé par le fait que le substrat est formé en employant la lactome-pyrrolidin-2-one (II)' et en ouvrant le noyau lactame.

5. Procédé selon la revendication 2, caractérisé par le fait qu'il comprend une condensation de GABA (II) avec de l'acide acrylique, ou un de ses esters, pour donner, respectivement, CEGABA (V) ou un de ses monoesters.

6. Procédé selon la revendication 2, caractérisé par le fait qu'il comprend l'estérification du GABA (II) pour donner un de ses esters (XI) et la condensation suivante avec de l'acide acrylique, ou un de ses esters, pour donner respectivement le monoester (IX) ou le diester (X) de CEGABA.

7. Procédé selon la revendication 3, caractérisé par le fait qu'il comprend la condensation de GABA (II) avec de l'acrylonitrile, suivie d'une hydrolyse pour donner CEGABA (V).

8. Procédé selon la revendication 1, caractérisé par le fait qu'il comprend une estérification de CEGABA (V) pour donner le diester correspondant de formule (I), dans laquelle $R_1$ et $R_2$ sont des alkyle.

9. Composés de formule (I) où $R_1$ et $R_2$ = H ou alkyle inférieur pour l'utilisation en vue de fournir une activité similaire au GABA dans le cerveau.

10. Composition pharmaceutique contenant un composé selon la revendication 9 apte à fournir une activité similaire au GABA dans le cerveau.

**Revendications pour l'Etat contractant: AT**

1. Procédé de préparation de composés possédant une activité anti-convulsivante, analogue au GABA, dans le cerveau, de formule générale:

$$R_1OOC—(CH_2)_2—NH—(CH_2)_3—COOR_2 \quad (I)$$

dans laquelle $R_1$ et $R_2$ = H ou alkyle inférieur, caractérisé par le fait qu'il comprend une condensation d'un substrat choisi parmi: l'acide gamma-aminobutirique (II) (ou GABA) ou ses esters, avec un réactif choisi parmi: acrylate, acide acrylique ou ses esters.

2. Procédé selon la revendication 1, caractérisé par le fait que, lorsque ledit réactif est acrylonitrile, ladite condensation est suivie par une hydrolyse pour obtenir lesdits composés de formule (I).

3. Procédé selon la revendication 1, caractérisé par le fait que le substrat est formé en employant la lactame-pyrrolidin-2-one (II)' et en ouvrant le noyau lactame.

4. Procédé selon la revendication 2, caractérisé par le fait qu'il comprend une condensation de GABA (II) avec de l'acide acrylique, ou un de ses esters, pour donner, respectivement, CEGABA (V) ou un de ses monoesters.

5. Procédé selon la revendication 2, caractérisé par le fait qu'il comprend l'estérification de GABA (II) pour donner un de ses esters (XI) et la condensation suivante avec de l'acide acrylique, ou un de ses esters, pour donner respectivement le monoester (IX) ou le diester (X) de CEGABA.

6. Procédé selon la revendication 2, caractérisé par le fait qu'il comprend la condensation de GABA (II) avec de l'acrylonitrile, suivie d'une hydrolyse pour donner CEGABA (V).

7. Procédé selon la revendication 1, caractérisé par le fait qu'il comprend une estérification de CEGABA (V) pour donner le diester correspondant de formule (I) dans laquelle $R_1$ et $R_2$ sont des alkyle.